Europäisches Patentamt

European Patent Office

Office européen des brevets

(11) Publication number: **0 244 100 B1**

# EUROPEAN PATENT SPECIFICATION

(45) Date of publication of patent specification: **27.03.91**  (51) Int. Cl.5: **B65D  25/10**, B01L 9/06

(21) Application number: **87302960.7**

(22) Date of filing: **06.04.87**

(54) Improvements in or relating to record containers.

(30) Priority: **26.04.86 GB 8610276**

(43) Date of publication of application:
**04.11.87 Bulletin  87/45**

(45) Publication of the grant of the patent:
**27.03.91 Bulletin  91/13**

(84) Designated Contracting States:
**AT BE CH DE ES FR GB GR IT LI LU NL SE**

(56) References cited:
**EP-A- 0 139 412
DE-A- 2 143 451
DE-A- 2 528 152
US-A- 3 301 619
US-A- 3 604 566**

(73) Proprietor: **MOORE BUSINESS FORMS, INC.
300 Lang Boulevard
Grand Island New York 14072-1697(US)**

(72) Inventor: **Fasham, Thomas Ian Kennedy
75/79 Southwark Street
London SE1 0HY(GB)**

(74) Representative: **Townsend, Derek Thomas et
al
Spence & Townsend Mill House Wandle
Road Beddington
Croydon Surrey CR0 4SD(GB)**

## Description

This invention has reference to record retainers and has particular but not exclusive reference to retainers for business forms records of transactions made in relation to medical records. For example the record retainers may be used to retain records of medical transactions as described for example in the specifications of our British Patent Applications Nos 8324568 and 8324569 (European Patent Applications Nos 84305877.7 and 84305878.5) and in particular to enable such medical records to be transported and/or stored and/or processed.

In the specification of British Application for Patent No 8324568 (European Patent Application No 84305877.7) there is described a business forms assembly comprising a medical form sheet with provision for receiving information about a medical test and a bag having closure means at the mouth of the bag to secure in the bag a container for receiving a medical test sample and wherein the bag is secured by adhesive to the business form sheet. Such business forms assemblies may be supported upon a bar having limbs, the lower limb of which is capable of supporting the business forms assembly by passing through an aperture in each assembly and the upper limb of which is formed as a carrying handle whereby each of the business forms assemblies may be supported.

Similarly in the specification of British Application for Patent No 8324569 EP-A-0 139 413 there is described a medical forms assembly which comprises a backing sheet with one part thereof having a transparent sheet secured thereto to provide a bag to contain a medical sample and another part thereof intended to receive information about the medical sample.

In the medical forms assemblies described in each of the above referred to specifications there is also described the feature of an aperture (or apertures) in the medical form so that the assembly may be suspended on a filing post consisting of a plurality of limbs one limb of which serves to support medical forms and another of which limbs serves as a carrying handle. It is further described in EP-A-0 139 413 that said record support can be retained in a container.

Also specification DE-A-2538152 describes a transport container with a sliding front part and provision for receiving a plurality of trays each with provision for retaining sample carriers. The container has slotted partitions and the trays have ribs on opposite sides in order that the tray can slide into the container.

Specification US No 3604566 describes a test tube holder comprising a U-shaped base with side walls and support members with apertures to receive the test tubes with locking means to secure the support members in assembled relationship with the base.

Specification DE 2143451 describes a cardboard box with an upper wall and an intermediate partition with cut out holes to receive and support test tubes and associated documents in separate cut outs.

US Specification 3301619 describes a utility box and particularly a fishing tackle box to receive a plurality of trays provided with runners to guide the trays into the box. The box has a pivoted cover which overlies the top portion of the box.

It is an object of the present invention to provide a record container.

It is another object of the present invention to provide a record container which will enable medical forms, samples and records and especially samples and records of medical transactions to be retained in a simple and convenient manner for transport, storage and processing purposes in a hygenic manner.

According to the present invention a record container includes a record support which comprises a continuous bar having a plurality of limbs the lower limb of which is capable of securing a plurality of medical forms, each of which has an aperture therein and an upper limb having a first part formed as a carrying handle and another part serving as a locating member characterised in that said container has a pivoted front cover part with a peripheral seal and a plurality of sleeves located in the upper part thereof which sleeves are mounted on a cross member affixed to the container having the sleeves spaced along the length of said cross member and with the sleeves directed towards the opening closed by the front cover part to enable said locating member part to be slid into a selected sleeve to be retained in the container.

A record container in accordance with the present invention will now be described by way of example with reference to the accompanying drawings wherein:-

Fig. I is a perspective view of a record container, and

Fig. 2 is a perspective view of a record container with the cover part opened,

Fig. 3 is a sectional side view of the record container and

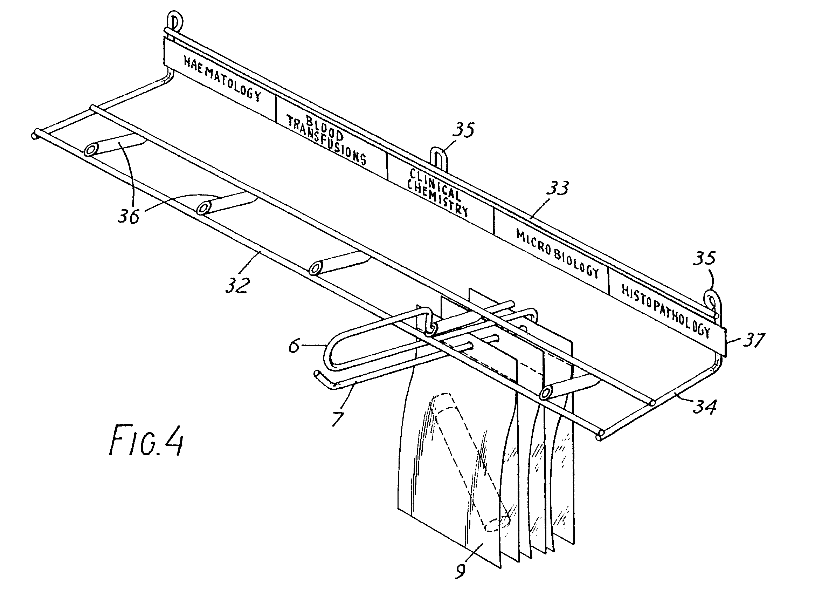

Fig. 4 is a view of an associated record support container.

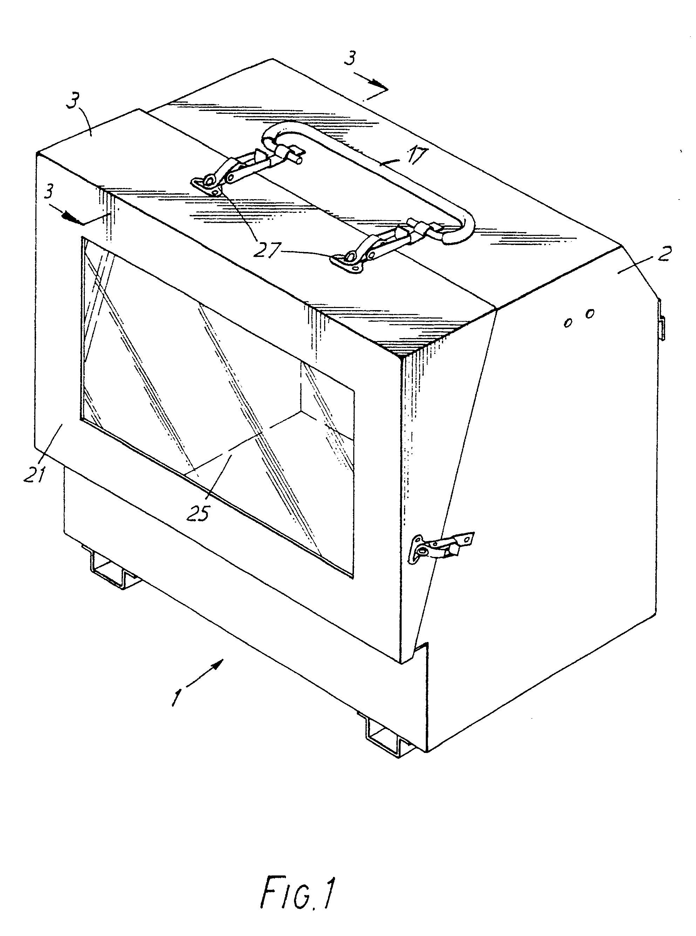

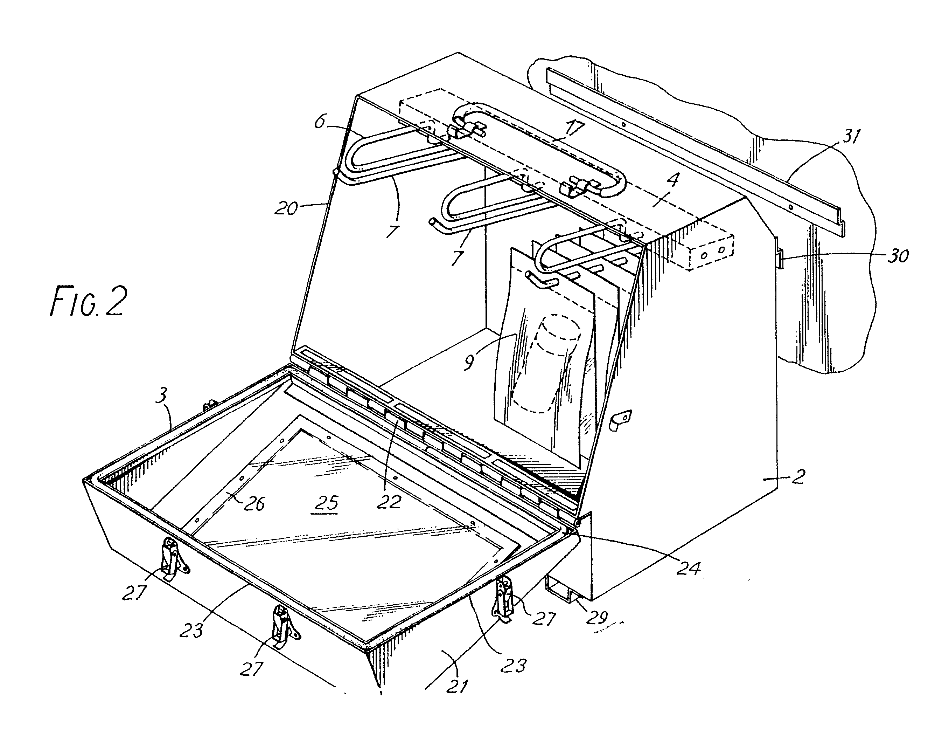

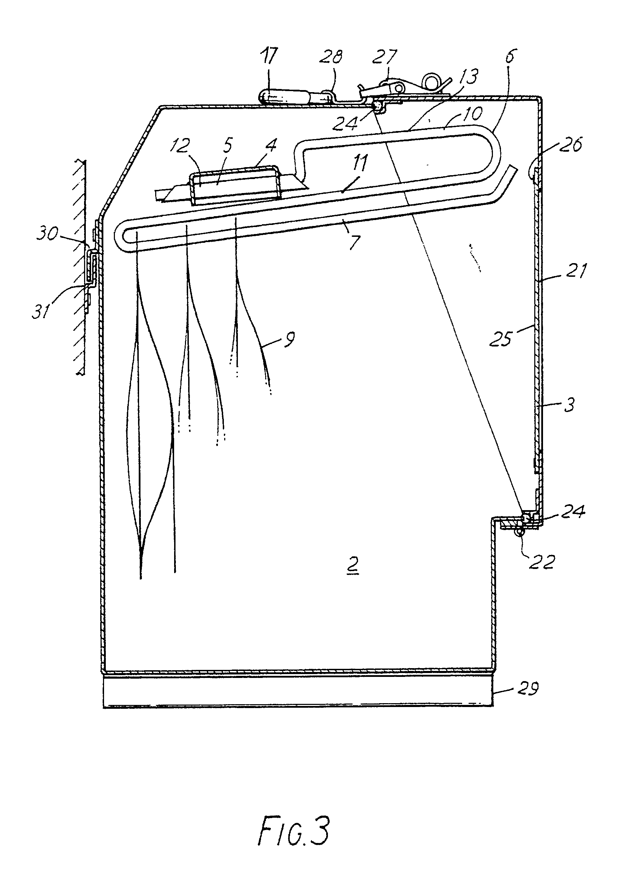

Referring to Figs. I and 2 of the drawings there is shown a box type record container I comprising a box part 2 with a pivotal front cover part 3 of transparent plastics material or anodised aluminium so as to be capable of being subjected to the conditions generated in an autoclave when the retainer is being cleaned and sterilized. The box part 2 has a transverse supporting cross frame member 4 secured to the box part 2 by bolts mounted between the sides of the box part 2. The cross member 4 is located in the roof of the box part 2 and bolted to the side walls of the box part 2 near to the rear of the box part. A plurality of sleeves 5 (three such sleeves are shown in Figs. 2 and 3) are secured as by welding to the cross member 4. As shown in Fig. 3 the sleeves 5 are so secured that the sleeves incline upwardly towards the front of the container.

The forward end of each sleeve 5 is chamfered to facilitate insertion of a complementary record support 6.

The front wall of the box part 2 of the container 1 has an opening 20 which is closed by a pivoted front cover member 21 which is pivoted on a hinge 22. This hinge has the hinge parts with a larger internal diameter than the outer diameter of the central rod passing through the hinge parts to provide a lost motion pivotal arrangement. The hinge is located adjacent the lower front end of the box part 2. The front member has a peripheral channel 23 around its inner face and a seal 24 of circular cross section of plastics or rubber material is located in this channel to render the fitting of the front cover member 21 in the box part 2 substantially waterproof. The frame part of the front member 21 is of aluminium and is capable of being subjected to autoclave conditions and has a transparent window 25 of polycarbonate material formed in it and secured as by a riveting frame 26 to the front cover member 21. An additional tray (not shown) may be provided within the base of the container 1 to enable the tray to be readily removed and cleaning operations of the retainer to be readily effected. A pair of toggle locks 27 are located on the upper face of the front cover member 21 to secure the pivoted front cover member securely. Similar toggle locks are provided on the sides of the front cover member 21. Each toggle lock 27 has a hasp and staple mechanism 28 to enable the lock to be secured by padlock.

The record support 6 comprises a continuous bar of metal for example of steel covered by a covering of plastics material for example polyethylene or polypropylene but preferably of nylon. The nylon covering may be applied by dipping the metal assembly in nylon powder or nylon preformer powder and is subsequently heat treated. The plastics material must be capable of withstanding steam cleaning and sterilizing as in an autoclave. The bar includes a lower limb 7 which is substantially straight and positioned horizontally but has an upwardly curved free end part 8. This lower limb 7 has a diameter slightly less than the internal diameter of the sleeves 5 and slightly less than the diameter of an aperture formed adjacent the upper edge of a medical form shown diagrammatically at 9 in Fig. 3 for example of the kind described in the specification of our application for Patent No. 8324568 or 8324569 (European Patent Applications Nos. 84305877.7 and 84305878.5) with which the record support 6 is to be associated. The forms 9 are capable of receiving a sample in a capsule or the like and of receiving information about the sample. The sample may be of blood, urine or other matter.

The bar also includes an upper limb 10 integrally connected to the lower limb 7 by an intermediate portion 11 adjacent its outer end the upper limb of which has a diameter the same as the diameter of the lower limb 7 and has a straight portion 12 which straight portion constitutes a locating member 12 so as to be located within one of the sleeves 5 as best shown in Fig. 3. The upper limb bends upwardly at about its mid point and to form a further substantially horizontal portion 13 which portion 13 constitutes a carrying handle part.

As shown it is intended that there are three sleeves secured to the cross member 4 with the sleeves so spaced so that when the medical forms 9 which when folded have a depth of approximately nine and one half (9½) inches (24 centimetres) and has a width of approximately five (5) inches (13 centimetres) the forms lie close to the base of the box part 2 and the forms are positioned in groups which groups lie close to one another side by side. These forms are of the kind which have a bag or pocket to receive a medical sample (for example a blood or urine sample) and the forms are arranged to receive information about the person concerned with the medical sample.

The box part 2 of the recorder container 1 has its rear and side parts and the lower portion of the front part and the rear portion of the upper part formed as a one piece box.

The box part 2 is preferably made of aluminium and the front cover member 21 is also made of aluminium. A carrying handle 17 is provided on the top of the box part 2. The handle is of metal covered with a sleeve of silicon rubber capable of withstanding autoclave conditions.

Each of the box parts 2 have a pair of spacer flanges 29 to retain the record container off the ground and serve as spacer stacking feet members to enable several record retainers to be stacked one upon the other for storage or transportation purposes. A flange 30 is provided on the rear face of the box part 2 and cooperates with a complementary flange part 31 secured as by screws to a wall. By mounting the flange 30 on the flange part 31 the record container 1 can be supported on a wall or like surface.

When the record container 1 is to be used for retaining medical records and samples a medical form 9 has medical data applied to it and is accompanied by a sample as described in the specification of European Patent Applications Nos. 84305877.7 and 84305878.5. One of the record supports 6 is removed from the supportive sleeve 5 in the record container I by pivoting open the front cover part 2I. The record support has its upper limb I0 slid out of the complementary sleeve 5 and removed from the box part 2. A medical form 9 or several of such forms 9 are slid on to the lower limb of the record support 6 and the record support 6 replaced in the box part 2 by sliding the upper limb back into the sleeve 5 and the front cover member 2I pivoted closed.

Several such medical forms may be placed on the record supports in the box part 2 as collated from for example different wards in a hospital and taken to an appropriate laboratory for sample test purpose. If desired one record container may be used for example to contain sample medical forms for a haematology test and others for microbiology and clinical chemistry. At the various laboratories further information about the various samples may be applied to the forms as appropriate.

It will be apparent that the box part 2, cover part 3 and the record support 6 are capable of being subjected to heat treatment such as steam cleaning as in an autoclave for sterilization purposes.

Conveniently a series of printed labels may be secured to the cross member 4 to identify the destination of samples and documentation secured on a record support 6.

To assist in the processing of samples and documentation a supporting sleeve member 32 is mounted as on a wall. The member 32 comprises a framework having three horizontally disposed bars 33 which are secured as by welding between a pair of side members 34. The rear horizontal bar has a set of eyelets 35 to enable the sleeve member to be mounted on a wall as by screws.

A plurality of sleeves 36 are secured as by welding to the front two horizontally disposed bars 33. These sleeves 36 are spaced along the bars 33 so that each sleeve is capable of receiving a record support 6 carrying a plurality of medical forms 9. The sleeves 36 are secured as by welding to the bars 33 and are so located that they slope downwardly towards the wall. These sleeve members 32 are located at a suitable location in or near a research laboratory or the like in a hospital so that when a porter is carrying a record container I (as shown in Figs. I to 3) he takes one of the record supports from the container I and inserts it in one of the sleeves 36 on the sleeve member 35 where the samples and documentation may be removed and processed further by the laboratory staff. A label bar 37 is attached between the side members 34 and labels identifying the destination of the samples and documentation (for example the Haematology laboratory, the blood transfusion laboratory) are attached to the label bar 37.

## Claims

1. A record container (1) including a record support (6) which comprises a continuous bar having a plurality of limbs the lower limb (7) of which is capable of securing a plurality of medical forms (3), each of which has an aperture therein, and an upper limb (10) having a first part formed as a carrying handle (13) and another part serving as a locating member (12) characterised in that said container has a pivoted front cover part (21) with a peripheral seal (24) and a plurality of sleeves (5) located in the upper part thereof which sleeves are mounted on a cross member (4) affixed to the container having the sleeves (5) spaced along the length of said cross member and with the sleeves (5) directed towards the opening (20) closed by the front cover part (21) to enable said locating member part (12) to be slid into a selected sleeve (5) to be retained in the container.

2. A record container as claimed in Claim 1 characterised in that the pivoted front cover part (21) contains a transparent window (25) secured to the cover part by a riveted frame.

3. A record container as claimed in Claims 2 or 3 characterised in that toggle locks (27) are located on the pivoted cover part (21) to secure said part securely in its closed position.

4. A record container as claimed in claim 1 wherein the sleeves (5) incline upwardly towards the opening (10) in the front of the container.

5. A record container as claimed in Claim 1 characterised in that the container has spaced flanges (29) on its bottom panel for stacking containers one upon the other.

6. A record container as claimed in any one of the preceding claims characterised in that a flange (30) is provided on the rear panel of the container and cooperates with a complementary flange (31) secured as by screws to a wall.

## Revendications

1. Conteneur à dossiers (1) comprenant un support de dossiers (6) qui est constitué par une barre continue ayant plusieurs branches, dont la branche inférieure (7) est adaptée à supporter plusieurs imprimés médicaux (3) dont chacun comporte une ouverture, et une branche supérieure (10) ayant une première partie formant une poignée de transport (13) et une autre partie servant d'organe de positionnement (12), caractérisé en ce que le conteneur comporte une partie articulée avant formant couvercle (2l) avec un joint périphérique (24), et plusieurs manchons (5) disposés dans la partie supérieure du conteneur, ces manchons étant montés sur un organe transversal (4) fixé au conteneur, lequel contient les manchons (5) espacés suivant la longueur dudit organe transversal, et dirigés vers l'ouverture (20) qui est fermée par la partie avant (21) formant couvercle, afin de permettre de glisser l'organe de positionnement (12) dans un manchon choisi (5), pour être conservé dans le conteneur.

2. Conteneur à dossiers suivant la revendication 1, caractérisé en ce que la partie articulée avant formant couvercle (21) comporte une fenêtre transparente (25) fixée sur le couvercle au moyen d'un cadre rivé.

3. Conteneur à dossiers suivant la revendication 2 ou 3, caractérisé en ce que des dispositifs de fermeture à genouillère (27) sont disposés sur la partie avant articulée (21) formant couvercle afin de fixer le couvercle de façon sûre dans sa position de fermeture.

4. Conteneur à dossiers suivant la revendication 1, dans lequel les manchons (5) sont inclinés vers le haut en direction de l'ouverture (10) dans la partie avant du conteneur.

5. Conteneur à dossiers suivant la revendication 1, caractérisé en ce qu'il comporte des patins espacés (29) sur son fond pour empiler des conteneurs les uns sur les autres.

6. Conteneur à dossiers suivant l'une quelconque des revendications précédentes, caractérisé en ce qu'une ferrure à rebord (30) est prévue sur le panneau arrière du conteneur et coopère avec une ferrure à rebord complémentaire (31) fixée sur un mur, par exemple au moyen de vis.

**Ansprüche**

1. Dokumentenbehälter (1) mit einem Dokumententräger (6), der eine durchgehende Stange mit einer Vielzahl von Schenkeln umfaßt, von denen der untere Schenkel (7) eine Vielzahl von je eine Aussparung aufweisenden medizinischen Vordrucken (3) zu halten vermag, und ein oberer Schenkel (10) einen als Tragehandhabe (13) ausgebildeten ersten Abschnitt und einen anderen, als Aufnahmeteil (12) dienenden Abschnitt hat, dadurch gekennzeichnet, daß der Behälter ein angelenktes Frontdeckelteil (21) mit einer Umfangsdichtung (24) und eine in seinem oberen Teil angeordnete Vielzahl von Hülsen (5) aufweist, die an einem am Behälter befestigten Querstück (4) angeordnet sind, wobei die Hülsen (5) in Längsrichtung des Querstücks beabstandet sind und gegen die vom Frontdeckelteil (21) verschlossene Öffnung (20) gerichtet sind, derart, daß der Aufnahmeabschnitteil (12) zwecks Haltung im Behälter in eine ausgewählte Hülse (5) einschiebbar ist.

2. Dokumentenbehälter nach Anspruch 1, dadurch gekennzeichnet, daß das angelenkte Frontdeckelteil (21) ein durchsichtiges Fenster (25) enthält, das am Deckelteil durch einen Nietrahmen befestigt ist.

3. Dokumentenbehälter nach den Ansprüchen 2 oder 3, dadurch gekennzeichnet, daß auf dem angelenkten Deckelteil (21) Gelenkverschlüsse (27) angeordnet sind, derart, daß das Deckelteil in seiner Schließstellung sicher gehalten ist.

4. Dokumentenbehälter nach Anspruch 1, bei dem die Hülsen (5) zur Öffnung (20) in der Behältervorderseite hin schräg nach oben gerichtet sind.

5. Dokumentenbehälter nach Anspruch 1, dadurch gekennzeichnet, daß der Behälter an seiner Bodenplatte mit Zwischenabstand angeordnete Flansche (29) zum Aufeinanderstapeln von Behältern aufweist.

6. Dokumentenbehälter nach einem der vorhergehenden Ansprüche, dadurch gekennzeichnet, daß auf der Rückwand des Behälters ein Flansch (30) vorgesehen ist und mit einem z.B. an eine Wand angeschraubten komplementären Flansch (31) zusammenwirkt.

FIG.1

FIG.2

EP 0 244 100 B1

FIG.3

FIG.4